# EUROPEAN PATENT APPLICATION

(11) **EP 4 471 124 A1**
(43) Date of publication of application: **04.12.2024**
(21) Application number: 23743339.6
(22) Date of filing: 20.01.2023
(51) Int. Cl.: C12N 5/076, C12N 5/0775, C12N 1/00

(54) **COMPOSITION FOR SPERM TREATMENT, SPERM MOTILITY IMPROVING AGENT, SPERM FERTILIZING CAPABILITY RETAINING AGENT, SPERM MOTILITY IMPROVING METHOD, AND SPERM FERTILIZING CAPABILITY RETAINING METHOD**

(30) Priority: 24.01.2022 JP 2022008745; 28.10.2022 JP 2022173214
(71) Applicant: Hiroshima University, Higashihiroshima-shi Hiroshima 739-8511 (JP); Rohto Pharmaceutical Co., Ltd., Osaka-shi, Osaka 544-8666 (JP)
(72) Inventor: SHIMADA Masayuki, Higashi-Hiroshima City, Hiroshima 739-8511 (JP); UMEHARA Takashi, Higashi-Hiroshima City, Hiroshima 739-8511 (JP); ISHIKAWA Noriyasu, Soraku-gun, Kyoto 619-0237 (JP); TAKIJIRI Takashi, Osaka-shi, Osaka 544-8666 (JP)
(74) Representative: Mewburn Ellis LLP
(86) International application number: PCT/JP2023/001660
(87) International publication number: WO 2023/140347

(57) **Abstract**

The purpose of the present invention is to provide sperm having high motility. The present invention is a composition for treating sperm that comprises medium for mesenchymal stem cells. This composition for treating sperm is used as a sperm preparing solution, a sperm diluting solution, a sperm storage solution, an artificial insemination solution, an in vitro fertilization solution, a solution for improving sperm motility, or a solution for retaining sperm fertilizing capabilities. Additionally, the medium for mesenchymal stem cells contained in the composition for treating sperm is preferably a serum-free medium.

## Description

### TECHNICAL FIELD

The present invention relates to a composition for treating sperm, an agent for improving sperm motility, an agent for retaining sperm fertility, a method for improving sperm motility, and a method for retaining sperm fertility.

### BACKGROUND ART

"Infertility" refers to the inability to conceive for a certain period of time despite sexual intercourse without contraception between a healthy man and woman who wish to conceive, and this "certain period of time" is defined as "generally one year" by the Japan Society of Obstetrics and Gynecology. It is estimated that about one in ten couples are infertile, but in recent years, the age at which people think about conception has been rising, and it is known that both men and women become less likely to become pregnant as they age, and thus it is said that infertility rate is even higher.

Infertility may be caused by the male, by the female, or both, or by unknown cause, but it is said that about half of all cases the cause is also on the male side. Examples of a known cause of such male infertility include a sexual dysfunction such as spermatogenesis dysfunction, obstruction of sperm transport, erectile dysfunction (ED), or intravaginal ejaculation dysfunction, and aging. In men, it is said that the quality of sperm gradually deteriorates from around the age of 35. As people have been getting married later in life in recent years, it is said that about 10% of men have a problem with their semen findings even before marriage.

Infertility treatment is carried out by selecting the optimal treatment method according to the cause. Major treatment methods include a timing method, an ovulation induction method, artificial insemination, and even an assisted reproductive technique such as in vitro fertilization. Artificial insemination (AIH) is a treatment method involving injecting sperm into the uterine cavity in order to deliver sufficient sperm necessary for fertilization to the ampulla of the fallopian tube, which is the site of fertilization. AIH is indicated for oligozoospermia (sperm concentration of 15 million/ml or less), asthenospermia (motility rate of 40% or less), sexual intercourse dysfunction, sperm cervical mucus incompatibility (failing the Huhner test), a case of anti-sperm antibody retention, and a case of unexplained infertility. However, it is said that the limit of AIH is a total motile sperm count after treating of 1 million to 5 million, and there are also many cases in which it is difficult to satisfy this limit. In addition, when frozen semen is used for artificial insemination, the motility of the sperm decreases because of freezing, and the conception rate cannot currently be said to be high. Therefore, development of a method for enhancing the motility of sperm is desired.

Mesenchymal stem cells are multipotent progenitor cells that were first isolated from bone marrow by Friedenstein (1982) (see Non Patent Document 1). It has been revealed that mesenchymal stem cells are present in various tissues such as bone marrow, umbilical cord, and fat, and mesenchymal stem cell transplantation is expected as a new therapeutic method for various intractable diseases (see Patent Documents 1 and 2). Recently, it has been known that cells having comparable functions are present in stromal cells in adipose tissue, placenta, umbilical cord, egg membrane, and the like. Therefore, mesenchymal stem cells are sometimes called mesenchymal stromal cells.

### PRIOR ART DOCUMENTS

### Patent Document

Patent Document 1: JP 2012-157263 A
Patent Document 2: JP 2012-508733 A

### Non Patent Document

Non Patent Document 1: Pittenger F. M. et al., Science 284, pp.143-147, 1999

### SUMMARY OF INVENTION

### Technical Problem

Under the circumstances described above, an object of the present invention is to provide sperm having excellent motility.

### Solution to Problem

The present inventors have carried out intensive research in order to solve the above problem and as a result, found that a medium for mesenchymal stem (stromal) cell (MSC) culture increases the motion performance of sperm, and completed the present invention. According to the present invention, sperm having high motility and suppressed sperm acrosome reaction can be provided. That is, the gist of the present invention is as follows.
[1] A composition for treating sperm, comprising a medium for mesenchymal stem cells.
[2] The composition for treating sperm according to [1], wherein the composition is a sperm conditioning solution, a sperm dilution solution, a sperm preservation solution, an artificial insemination solution, an in vitro fertilization solution, a solution for improving sperm motility, or a solution for retaining sperm fertility.
[3] The composition for treating sperm according to [1] or [2], wherein the medium for mesenchymal stem cells is a serum-free medium.
[4] The composition for treating sperm according to [3], wherein the medium for mesenchymal stem cells comprises at least one selected from the group consisting of EGF, bFGF, albumin, transferrin, and insulin.
[5] An agent for improving sperm motility, comprising a medium for mesenchymal stem cells.
[6] An agent for retaining sperm fertility, comprising a medium for mesenchymal stem cells.
[7] A method for improving sperm motility, comprising culturing sperm in a solution comprising a medium for mesenchymal stem cells.
[8] A method for retaining sperm fertility, comprising culturing sperm in a solution comprising a medium for mesenchymal stem cells.

### Advantageous Effects of Invention

According to the present invention, sperm having high motility and suppressed sperm acrosome reaction can be provided. Sperm treated by the present invention have higher motility than and suppressed sperm acrosome reaction as compared with sperm treated by a conventional method, and thus have a remarkably higher fertilization success rate.

### BRIEF DESCRIPTION OF DRAWINGS

[Fig. 1] Fig. 1 is a diagram showing the motility of mouse sperm under each condition.
[Fig. 2] Fig. 2 is a diagram showing results of counting sperm acrosome marker positive cells under each condition by flow cytometry.
[Fig. 3] Fig. 3 is a diagram showing the proportion of acrosome marker positive sperm under each condition.
[Fig. 4] Fig. 4 is a diagram showing the motility of mouse sperm under each condition.
[Fig. 5] Fig. 5 is a diagram showing the sperm acrosome retention rate of mouse sperm under each condition.
[Fig. 6] Fig. 6 is a diagram showing the cleavage rate under each condition.
[Fig. 7] Fig. 7 is a histogram showing the progressive motility of cattle sperm under each condition.
[Fig. 8] Fig. 8 is a histogram showing the curvilinear motility of cattle sperm under each condition.
[Fig. 9] Fig. 9 is a histogram showing the amplitude of lateral head displacement of cattle sperm under each condition.
[Fig. 10] Fig. 10 is a diagram showing the extent of increase in the median value of the straight-line velocity of pig sperm.
[Fig. 11] Fig. 11 is a diagram showing the proportion of motile sperm in all sperm observed for pig sperm under each condition.
[Fig. 12] Fig. 12 is a diagram showing the straight-line velocity of pig sperm under each condition.

### DESCRIPTION OF EMBODIMENTS

Hereinafter, the composition for treating sperm, the agent for improving sperm motility, the agent for retaining sperm fertility, the method for improving sperm motility, and the method for retaining sperm fertility according to the present invention will be described in detail.

### [Composition for treating sperm]

The composition for treating sperm according to the present invention is characterized by including a medium for mesenchymal stem cells. The composition for treating sperm according to the present invention can provide sperm having high motility and suppressed sperm acrosome reaction by including a medium used to culture mesenchymal stem cells. Sperm treated with the composition for treating sperm according to the present invention and thereby having improved or increased motility and suppressed acrosome reaction can be expected to, for example, increase fertilization efficiency or conception rate in artificial insemination or in vitro fertilization, or promote early development.

The motility of sperm can be expressed by a sperm motion parameter. Examples of such a motion parameter include the amplitude of the lateral oscillatory movement of the sperm head (amplitude of lateral head displacement; ALH), the number of lateral oscillatory movements of the sperm head (beat cross frequency; BCF), the velocity based on the total distance traveled by sperm (curvilinear velocity; VCL), and the velocity based on the straight-line distance traveled by sperm (straight-line velocity; VSL). ALH/VCL (value obtained by dividing ALH by VCL) indicates the degree of motility in zigzag movement characteristic of sperm. In addition, BCF/VSL (value obtained by dividing BCF by VSL) indicates the degree of motility in straight-line movement of sperm.

The medium for mesenchymal stem cells included in the composition for treating sperm according to the present invention may be any medium as long as the medium is a medium that allows mesenchymal stem cells to proliferate while maintaining the ability to differentiate into osteocytes, chondrocytes, and adipocytes, and is not particularly limited.

The medium for mesenchymal stem cells used in the present invention may be created by supplementing a basal medium with a serum and/or with one or more serum substitutes such as albumin, transferrin, insulin, sodium selenite, cholesterol, a collagen precursor, a trace element, 2-mercaptoethanol, or 3'-thiolglycerol. In addition, if necessary, the medium for mesenchymal stem cells used in the present invention may be further supplemented with a substance such as an amino acid such as glutamine, a sugar such as glucose, a metal salt such as sodium chloride or magnesium sulfate, a trace metal such as selenium, a vitamin such as pantothenic acid, a protein such as albumin, insulin, transferrin, a growth factor, a proliferation factor, or a cytokine, a polysaccharide, a low-molecular-weight compound, an antibiotic, an antioxidant, a pyruvic acid, a buffer, or an inorganic salt.

Examples of the basal medium include IMDM medium, Medium 199 medium, Eagle's Minimum Essential Medium (EMEM) medium, αMEM medium, Dulbecco's modified Eagle's Medium (DMEM) medium, Ham's F12 medium, RPMI 1640 medium, Fischer 's medium, MCDB201 medium, and a mixed medium thereof.

From the viewpoint of use for treating sperm, the medium for mesenchymal stem cells is preferably a xeno-free medium, which is free of a xeno-derived component such as a serum. Examples of such a medium include a medium provided as a pre-prepared medium for mesenchymal stem cells (stromal cells) such as Mesenchymal Stem Cell Growth Medium 2 (Ready-to-use, manufactured by PromoCell), Mesenchymal Stem Cell Growth Medium XF (Ready-to-use, manufactured by PromoCell), MSCGM BulletKittm, MSCGMtm Mesenchymal Stem Cell Growth Medium BulletKittm (manufactured by Lonza), a xeno-free medium for human mesenchymal stem cells (MSC NutriStem (registered trademark) XF, manufactured by Biological Industries), MesenCult-ACF Plus (manufactured by Veritas), StemXVivotm Serum-Free Human MSC Expansion Media (manufactured by R&D Systems, Corning), a serum-free medium for adipose-derived stem cells (KBM ADSC-4, manufactured by Kohjin Bio), and a serum-free medium for mesenchymal stem cells (R: STEM Medium for hMSC High Growth, manufactured by Rohto).

Examples of the serum described above include, but are not limited to, human serum, fetal bovine serum (FBS), bovine serum, calf serum, goat serum, horse serum, pig serum, sheep serum, rabbit serum, and rat serum. When a serum is used, a basal medium may be supplemented with 5 v/v% to 15 v/v%, preferably 10 v/v%, thereof.

Examples of the amino acid described above include, but are not limited to, L-alanine, L-arginine, L-aspartic acid, L-asparagine, L-cysteine, L-cystine, L-glutamic acid, L-glutamine, and L-glycine. Examples of the protein include, but are not limited to, ecotin, reduced glutathione, fibronectin, and β2-microglobulin. Examples of the polysaccharide include, but are not limited to, a glycosaminoglycan, and particular examples of the glycosaminoglycan include, but are not limited to, hyaluronic acid and heparan sulfate. Examples of the proliferation factor include, but are not limited to, platelet-derived growth factor (PDGF), basic fibroblast growth factor (bFGF), transforming growth factor beta (TGF-β), hepatocyte growth factor (HGF), epidermal growth factor (EGF), connective tissue growth factor (CTGF), and vascular endothelial growth factor (VEGF).

The composition for treating sperm according to the present invention preferably contains a medium for mesenchymal stem cells including at least one selected from the group consisting of EGF, bFGF, albumin, transferrin, and insulin, more preferably a medium for mesenchymal stem cells including at least two selected therefrom, further preferably a medium for mesenchymal stem cells including at least three selected therefrom, and particularly preferably a medium for mesenchymal stem cells including at least four selected therefrom, in addition to a basal medium, and most preferably contains a medium for mesenchymal stem cells including EGF, bFGF, albumin, transferrin, and insulin in addition to a basal medium.

The medium for mesenchymal stem cells contained in the composition for treating sperm according to the present invention is preferably free of a surfactant. The surfactant is not particularly limited, and examples thereof include Pluronic F-68 and polyoxyethylene sorbitan monooleate (Tween 80). If the medium for mesenchymal stem cells contained in the composition for treating sperm according to the present invention includes a surfactant, it becomes difficult to obtain the following effect of the present invention: sperm having high motility and suppressed sperm acrosome reaction can be provided. In order to dissolve a lipid component in the medium for mesenchymal stem cells, it is necessary to add a surfactant, and thus, the medium for mesenchymal stem cells contained in the composition for treating sperm according to the present invention is preferably free of a lipid component such as cholesterol, a saturated fatty acid, or an unsaturated fatty acid.

The amount of the medium for mesenchymal stem cells included in the composition for treating sperm according to the present invention is 0.1% by weight to 100% by weight, preferably 0.5% by weight to 95% by weight, more preferably 1% by weight to 90% by weight, further preferably 3% by weight to 80% by weight, and particularly preferably 5% by weight to 50% by weight, of the whole composition for treating sperm. By setting the amount of the medium for mesenchymal stem cells included in the composition for treating sperm according to the present invention within the above numerical range, the effect of increasing the motility of sperm and suppressing the acrosome reaction of sperm while suppressing damage to the sperm is excellent.

The composition for treating sperm according to the present invention may contain a further component as long as the further component does not impair the effect of the present invention, in addition to the medium for mesenchymal stem cells. Examples of the further component include a protective agent such as dimethylsulfoxide (DMSO) or serum albumin, an antibiotic, a vitamin, a carrier, an excipient, a disintegrant, a buffer, an emulsifier, a stabilizer, a preservative, an antiseptic, and physiological saline. In addition, a buffer solution that is commercially available as a buffer solution (washing solution) for an ovum or sperm or the like may be used. Preferred examples of such a buffer solution include HTF Medium (manufactured by FUJIFILM Wako Pure Chemical Corporation) containing NaCl, KCl, KH₂PO₄, MgSO₄ ·7H₂O, CaCl₂ ·2H₂O, NaHCOs, Glucose, Na-pyruvate, Na-lactate, Phenol Red, Hepes, Gentamicin Sulfate salt, and the like.

The species of sperm that are the target of the composition for treating sperm according to the present invention may be any mammalian animal, and examples thereof include a human, a horse, cattle, a sheep, a pig, a dog, a cat, a rabbit, a mouse, a rat, and a rare animal.

The composition for treating sperm according to the present invention is used as a sperm conditioning solution, a sperm dilution solution, a sperm preservation solution, an artificial insemination solution, an in vitro fertilization solution, a solution for improving sperm motility, a solution for retaining sperm fertility, or the like.

The sperm conditioning solution is a solution for washing sperm and even suspending sperm to recover sperm that show good motility, when providing sperm for the purpose of artificial insemination, in vitro fertilization, or the like. The sperm dilution solution is a solution used to dilute sperm to an appropriate concentration and inject the same into the uterus when carrying out artificial insemination. The sperm preservation solution is a solution that can be used for chilled preservation or frozen preservation of sperm. The artificial insemination solution is a solution used to increase the fertilization rate in artificial insemination. The in vitro fertilization solution is a solution used to increase the fertilization rate in in vitro fertilization. The solution for improving sperm motility is a solution used to improve the motility of sperm. The solution for retaining sperm fertility is a solution used to retain the fertility of sperm.

The composition for treating sperm according to the present invention can be prepared by a conventional method after mixing a further necessary component with the medium for mesenchymal stem cells described above.

### [Agent for improving sperm motility and agent for retaining sperm fertility]

The agent for improving sperm motility and the agent for retaining sperm fertility according to the present invention are characterized by including a medium for mesenchymal stem cells. The agent for improving sperm motility and the agent for retaining sperm fertility according to the present invention can provide sperm having increased sperm motility and suppressed acrosome reaction, by including a medium used to culture mesenchymal stem cells. Sperm treated with the agent for improving sperm motility or the agent for retaining sperm fertility according to the present invention can be expected to increase fertilization efficiency and conception rate in artificial insemination or in vitro fertilization, promote early development, or the like.

The agent for improving sperm motility and the agent for retaining sperm fertility according to the present invention are substantially the same as the composition for treating sperm described above, and thus for a specific description thereof, the description in the section Composition for treating sperm can be applied as it is by replacing the composition for treating sperm with the agent for improving sperm motility or the agent for retaining sperm fertility.

In a method for treatment with the agent for improving sperm motility and the agent for retaining sperm fertility according to the present invention, a state in which the agent for improving sperm motility or the agent for retaining sperm fertility is in contact with sperm is formed in vivo or in vitro. In vivo, it is considered to administer the agent for improving sperm motility or the agent for retaining sperm fertility to the seminal vesicle, which is an organ for storing sperm. As long as a state in which the agent for improving sperm motility or the agent for retaining sperm fertility according to the present invention is in contact with sperm is formed after administration, the administration site and the administration method are not particularly limited. On the other hand, in order to in vitro form a state in which the agent for improving sperm motility or the agent for retaining sperm fertility according to the present invention is in contact with sperm, for example, previously provided sperm and the agent for improving sperm motility or the agent for retaining sperm fertility according to the present invention are placed and mixed in an appropriate container in vitro and treated for a certain period of time. The treatment temperature is 4°C to 40°C, preferably 15°C to 40°C, more preferably 25°C to 39°C, and further preferably 36°C to 38°C, which is about the same as body temperature. The treatment time is 10 minutes to 48 hours, preferably 20 minutes to 24 hours, more preferably 30 minutes to 4 hours, and further preferably 1 hour to 2 hours.

Sperm treated with the agent for improving sperm motility or the agent for retaining sperm fertility according to the present invention can be used for artificial insemination or in vitro fertilization (increased success rate or efficiency), breeding of livestock (increased success rate or efficiency of artificial insemination, breeding or maintenance of species (for example, maintenance of endangered species, or maintenance or crossbreeding of a pet strain), treatment or improvement of various diseases associated with sperm disorder or caused mainly or secondarily by sperm disorder (for example, varicocele, male infertility, cryptorchism, X-ray irradiation, or sperm disorder after malignant tumor surgery or chemotherapy), preservation of sperm, improvement of the motility, retention of the fertility, or preservation of immature sperm, and the like.

When artificial insemination is carried out by using sperm treated with the agent for improving sperm motility or the agent for retaining sperm fertility according to the present invention (also including artificial insemination for the purpose of breeding of livestock, or breeding or maintenance of species), a state in which sperm having improved motility and/or retained fertility and an ovum (egg cell, egg) coexist is formed in vivo or in vitro. In order to form the coexistence state in vivo, sperm having improved motility and/or sperm having retained fertility are administered to (implanted into) a site in a living organism where an ovum is present. For example, in the case of a mammalian animal, sperm treated with the present agent are injected into the uterus. On the other hand, if the coexistence state is formed in vitro, an ovum provided, for example, by isolation from a living organism or distribution from a cell bank or the like and sperm treated with the present agent may be placed in the same container and incubated, or sperm having improved motility and/or sperm having retained fertility may be inserted into the ovum by using a micromanipulator or the like. A conventional method can be followed except for using the condition characteristic of the present invention, that is, using sperm treated with the agent for improving sperm motility or the agent for retaining sperm fertility according to the present invention.

### [Method for improving sperm motility and method for retaining sperm fertility]

The present invention also includes a method for improving sperm motility and a method for retaining sperm fertility, characterized by culturing sperm in a solution including a medium for mesenchymal stem cells. In the method for improving sperm motility and the method for retaining sperm fertility according to the present invention, by culturing sperm in a solution including a medium used for culturing mesenchymal stem cells, the motility of the sperm can be improved and/or increased or the fertility of the sperm can be retained, while maintaining the acrosome. Sperm having motility increased and acrosome reaction suppressed by the method for improving sperm motility or the method for retaining sperm fertility according to the present invention can be expected to, for example, increase fertilization efficiency or conception rate in artificial insemination or in vitro fertilization, or promote early development.

The method for improving sperm motility and the method for retaining sperm fertility according to the present invention are methods for treating sperm with the agent for improving sperm motility or the agent for retaining sperm fertility according to the present invention described above, and thus for a specific description thereof, the section Agent for improving sperm motility and agent for retaining sperm fertility can be referred to.

### EXAMPLES

Hereinafter, the present invention will be described in detail with reference to Examples and Test Examples, but the present invention is not limited by these Examples and the like.

### [Example 1: Motility of sperm]

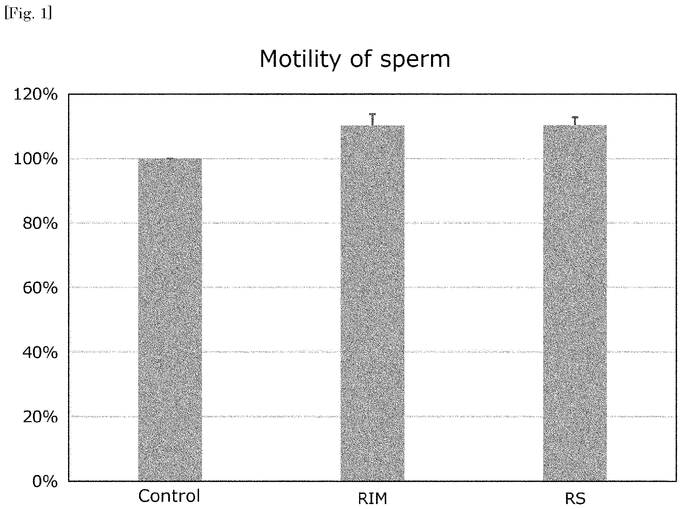

Mouse sperm (C57/BL6, 12 weeks old) were suspended in HTF Medium (manufactured by FUJIFILM Wako Pure Chemical Corporation) supplemented with RIM medium (serum-free medium for mesenchymal stem cells, Rohto, containing EGF, bFGF, albumin, transferrin, and insulin) or RS medium (serum-free medium for mesenchymal stem cells, Rohto, containing EGF, bFGF, albumin, transferrin, and insulin) in such a way as to have a final concentration of 10% (hereinafter also referred to as 10% RIM medium or 10% RS medium, respectively) and HTF Medium supplemented with no medium as a negative control (control) in a water bath at 37°C, and these were each used as a sample. Subsequently, the sample was cultured for 1 hour at 37°C. Next, 3 µL of the sample was analyzed by using a sperm motility analyzer (Computer Assisted Sperm Analysis (CASA) system). The straight-line velocity (VSL) of all sperm observed was analyzed. With the median value for the control set to 100, the median values for 10% RIM medium (RIM) and 10% RS medium (RS) are shown in Fig. 1.

As shown in Fig. 1, it was confirmed that the supplementation with the mesenchymal stem cell medium (RIM: 10% RIM medium, RS: 10% RS medium) increased the median value and increased the motility of the sperm.

### [Example 2: Suppression of acrosome reaction of sperm]

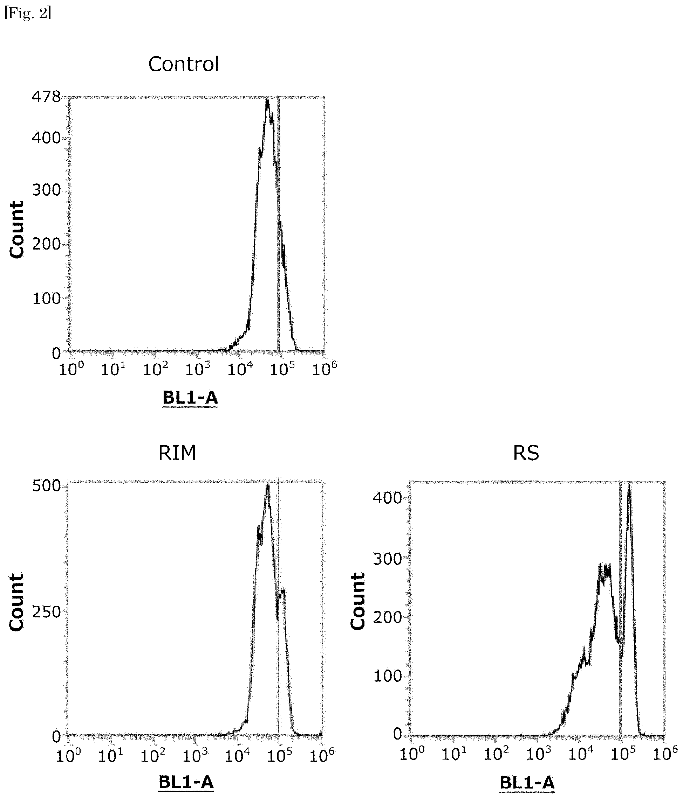

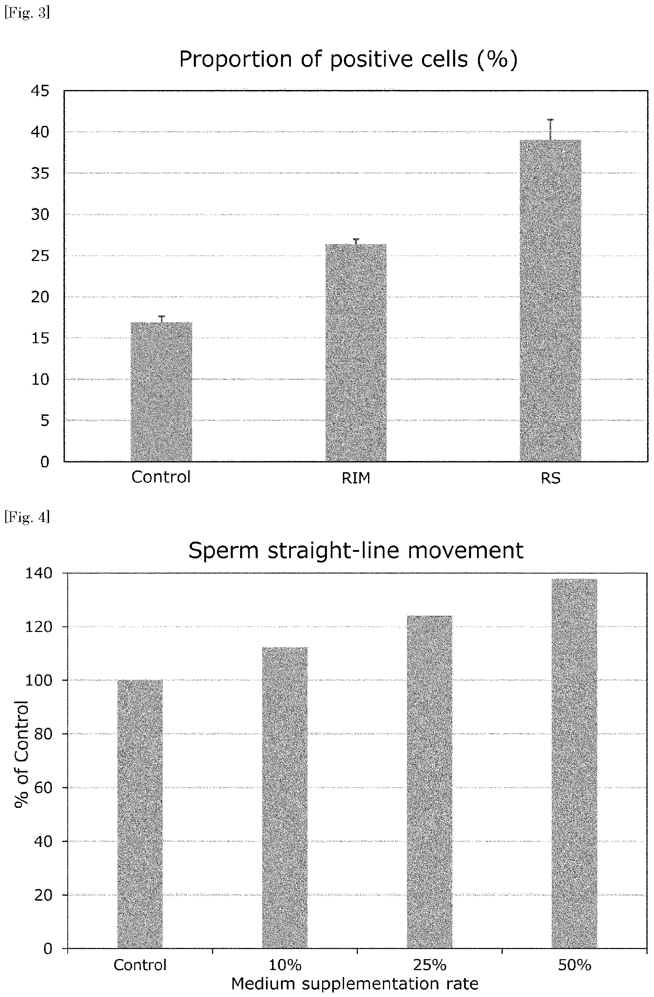

Sperm were collected from the epididymis of a male mouse (C57/BL6, 12 weeks old) and suspended in HTF Medium (manufactured by FUJIFILM Wako Pure Chemical Corporation) supplemented with RIM medium (serum-free medium for mesenchymal stem cells, Rohto) or RS medium (serum-free medium for mesenchymal stem cells, Rohto) in such a way as to have a final concentration of 10% and HTF Medium supplemented with no medium as a negative control, and after culture for 60 minutes, the proportion of sperm retaining an acrosome was analyzed by counting positive sperm by flow cytometry. Histograms of the number of positive sperm retaining an acrosome are shown in Fig. 2, and the proportions of the positive sperm are shown in Fig. 3 (RIM: 10% RIM medium, RS: 10% RS medium).

As shown in Fig. 2 and Fig. 3, it was confirmed that the supplementation with the medium for mesenchymal stem cells suppressed the acrosome reaction (RIM, RS). Therefore, it was revealed that the loss of fertility of sperm can be suppressed by supplementing a sperm medium such as HTF Medium with a medium for mesenchymal stem cells such as RIM medium or RS medium.

### [Example 3: Motility of sperm]

Mouse sperm (C57/BL6, 12 weeks old) were suspended in HTF Mediums (manufactured by FUJIFILM Wako Pure Chemical Corporation) supplemented with Lipid Free RIM medium (serum-free medium for mesenchymal stem cells, Rohto, containing EGF, bFGF, albumin, transferrin, and insulin and free of a fat component such as cholesterol, a saturated fatty acid, or an unsaturated fatty acid and a surfactant such as Pluronic F-68 or polyoxyethylene sorbitan monooleate (Tween 80)) in such a way as to have final concentrations of 10%, 25%, and 50% (hereinafter also referred to as 10%, 25%, and 50% Lipid Free RIM mediums, respectively) and HTF Medium supplemented with no medium as a negative control (control) in a water bath at 37°C, and these were each used as a sample. Subsequently, the sample was cultured for 1 hour at 37°C. Next, 3 µL of the sample was analyzed by using a sperm motility analyzer (Computer Assisted Sperm Analysis (CASA) system). The straight-line velocity (VSL) of all sperm observed was analyzed. With the median value for the control set to 100, the median values for 10%, 25%, and 50% Lipid Free RIM mediums are shown in Fig 4.

As shown in Fig. 4, it was confirmed that the supplementation with the mesenchymal stem cell medium (Lipid Free RIM medium) increased the median value and increased the motility of the sperm.

### [Example 4: Suppression of acrosome reaction of sperm]

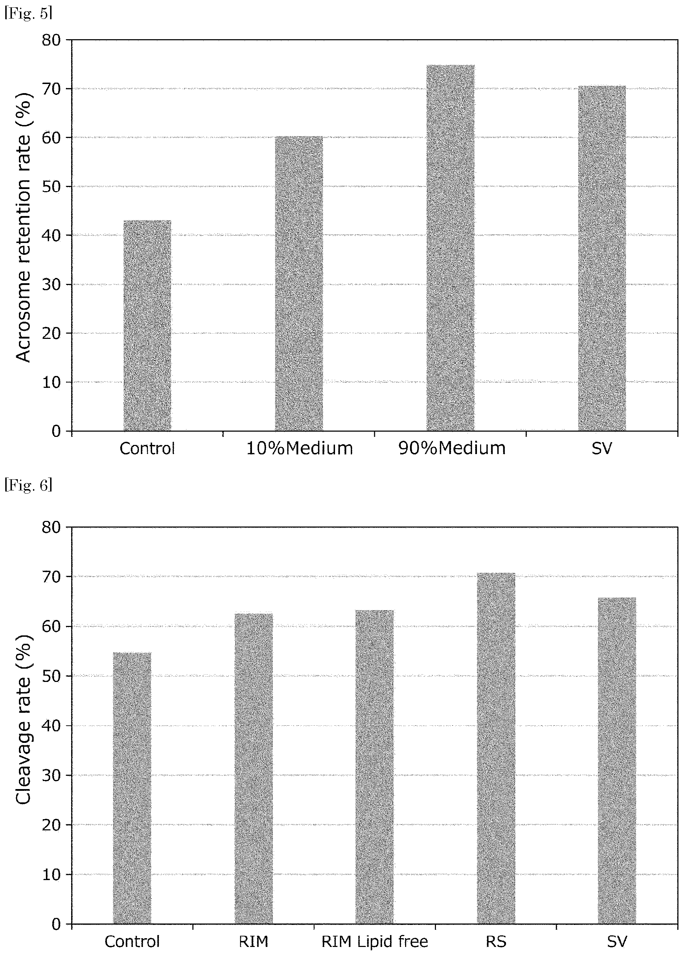

Sperm were collected from the epididymis of a male mouse (C57/BL6, 12 weeks old). HTF Mediums (manufactured by FUJIFILM Wako Pure Chemical Corporation) supplemented with Lipid Free RIM medium (serum-free medium for mesenchymal stem cells, Rohto) in such a way as to have final concentrations of 10% and 90% (hereinafter also referred to as 10% and 90% Lipid Free RIM mediums, respectively) and HTF Medium supplemented with no medium as a negative control (control) in which the sperm were suspended, and a seminal vesicle gland fluid (fluid obtained by homogenizing the seminal vesicle gland in HTF Medium and containing 10 mg/mL of protein (hereinafter also referred to as "sv")) as a positive target in which the sperm were suspended were cultured for 60 minutes, and then the proportion of sperm retaining an acrosome was analyzed by counting positive sperm by flow cytometry. Histograms of the number of positive sperm retaining an acrosome are shown in Fig. 5.

As shown in Fig. 5, it was confirmed that the supplementation with the Lipid Free medium for mesenchymal stem cells suppressed the acrosome reaction. Therefore, it was revealed that the loss of fertility of sperm can be suppressed by supplementing a sperm medium such as HTF Medium with a Lipid Free medium for mesenchymal stem cells.

### [Example 5: Fertilized egg acquisition rate]

Mouse sperm (C57/BL6, 12 weeks old, 1.0 × 10⁶ cells/mL) was suspended in HTF Medium (manufactured by FUJIFILM Wako Pure Chemical Corporation) supplemented with RIM medium (serum-free medium for mesenchymal stem cells, Rohto, containing EGF, bFGF, albumin, transferrin, and insulin), Lipid Free RIM medium (serum-free medium for mesenchymal stem cells, Rohto, containing EGF, bFGF, albumin, transferrin, and insulin and free of a fat component such as cholesterol, a saturated fatty acid, or an unsaturated fatty acid and a surfactant such as Pluronic F-68 or polyoxyethylene sorbitan monooleate (Tween 80)), or RS medium (serum-free medium for mesenchymal stem cells, Rohto, containing EGF, bFGF, albumin, transferrin, and insulin) in such a way as to have a final concentration of 10% (hereinafter also referred to as 10% RIM medium, 10% Lipid Free RIM medium, or 10% RS medium, respectively) and HTF Medium supplemented with no medium as a negative control (control), and these were each used as a sample and then allowed to stand in an incubator (37°C) for 30 minutes or more after preparing solution. In addition, mouse sperm were suspended in a seminal vesicle gland fluid (fluid obtained by homogenizing the seminal vesicle gland in HTF Medium and containing 10 mg/mL of protein) as a positive target, and this was used as a sample and then allowed to stand in an incubator (37°C) for 30 minutes or more after preparing solution. 40 µL of these cultured sperm suspensions were injected into the uterus of 7-week-old female mice, respectively, induced to estrus by exogenous hormone administration, by using a gel loading tip. Twenty-four hours after injection of the sperm suspensions, fertilized embryos were collected from the fallopian tubes of the female mice, and the proportion of 2-cell stage embryos was measured.

As shown in Fig. 6, it was confirmed that the treatment with the mesenchymal stem cell medium (10% RIM medium, Lipid Free RIM medium, 10% RS medium) increased the cleavage rate. It is considered that the increase in the progressive motility of sperm and the retention rate of the acrosome increased the acquisition rate of a fertilized egg.

### [Example 6: Motility of cattle sperm]

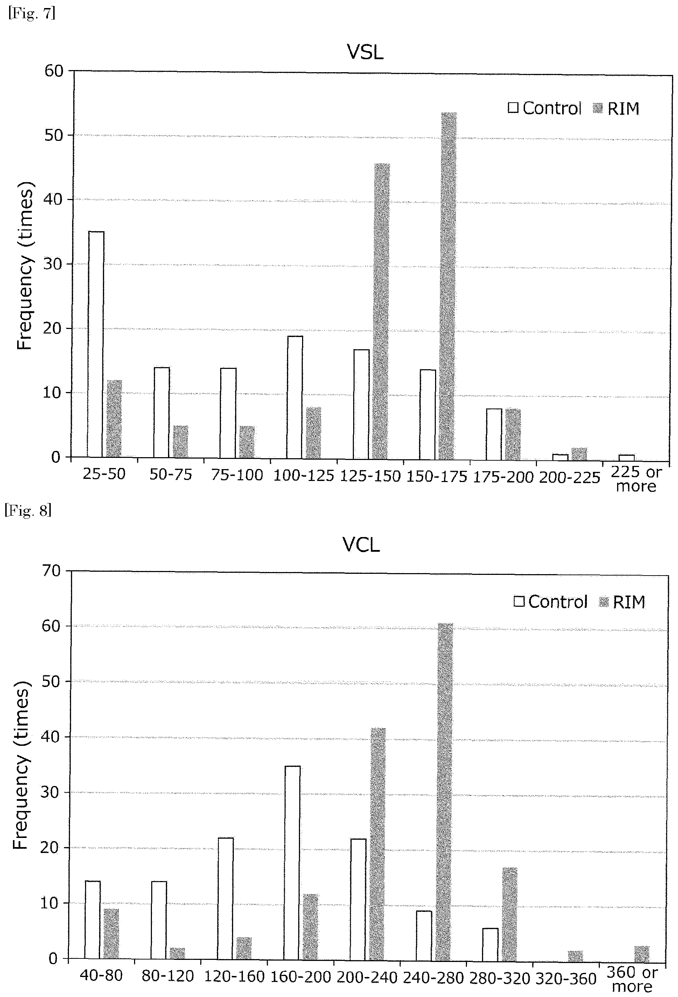

Fresh cattle sperm diluted with an egg yolk sodium citrate solution were washed twice with HTF Medium (manufactured by FUJIFILM Wako Pure Chemical Corporation). The washed sperm were suspended in HTF Medium supplemented with RIM medium (serum-free medium for mesenchymal stem cells, Rohto, containing EGF, bFGF, albumin, transferrin, and insulin) in such a way as to have a final concentration of 10% (10% RIM medium) and HTF Medium supplemented with no medium as a negative control (control), and these were each used as a sample. Subsequently, each sample was cultured for 1 hour at 37°C. Next, 3 µL of each sample was analyzed by using a sperm motility analyzer (Computer Assisted Sperm Analysis (CASA) system). The proportion of motile sperm in all sperm observed (Motility), and the straight-line velocity (VSL), the curvilinear velocity (VCL)), and the amplitude of lateral head displacement (ALH) thereof were analyzed (Fig. 7, Fig. 8, and Fig. 9).

It was confirmed that the supplementation with the mesenchymal stem cell medium also increased the motility of the sperm in the cattle, as in the mouse.

### [Example 7: Motility of pig sperm (1)]

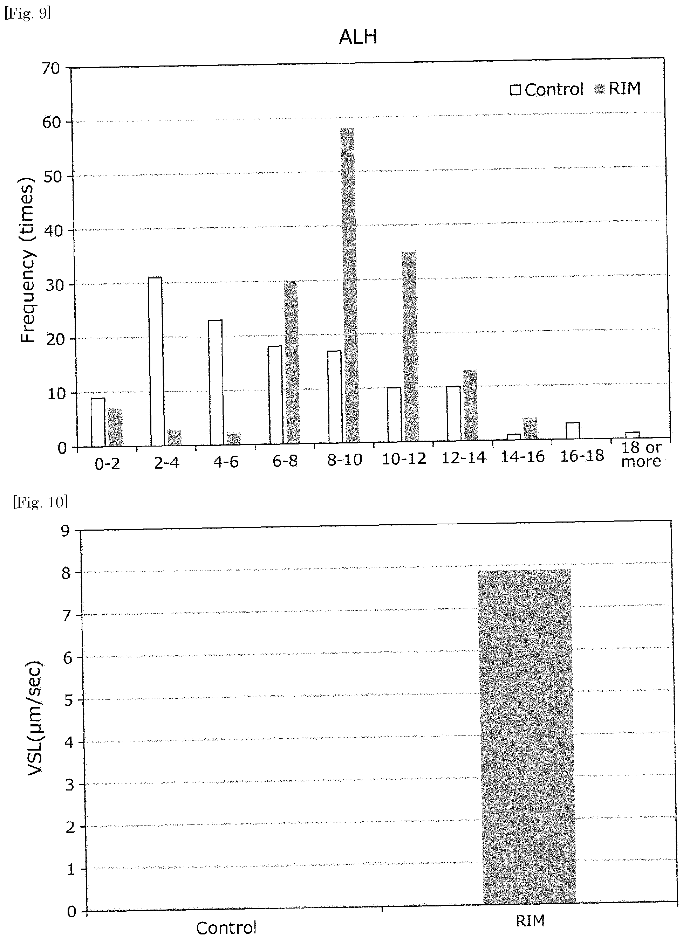

Fresh pig sperm the day after ejaculation diluted with HIRO-SWINE B Solution were washed twice with HTF Medium (manufactured by FUJIFILM Wako Pure Chemical Corporation). The washed sperm were suspended in HTF Medium supplemented with RIM medium (serum-free medium for mesenchymal stem cells, Rohto, containing EGF, bFGF, albumin, transferrin, and insulin) in such a way as to have a final concentration of 10% (10% RIM medium) and HTF Medium supplemented with no medium as a negative control (control), and these were each used as a sample. Subsequently, the sample was cultured for 1 hour at 37°C. Next, 3 µL of the sample was analyzed for straight-line velocity (VSL) by using a sperm motility analyzer (Computer Assisted Sperm Analysis (CASA) system), and the extent of increase in the median value for 10% RIM medium based on the median value for the control is shown in Fig. 10.

It was confirmed that the supplementation with the mesenchymal stem cell medium also increased the motility of the sperm in the pig.

### [Example 8: Motility of pig sperm (2)]

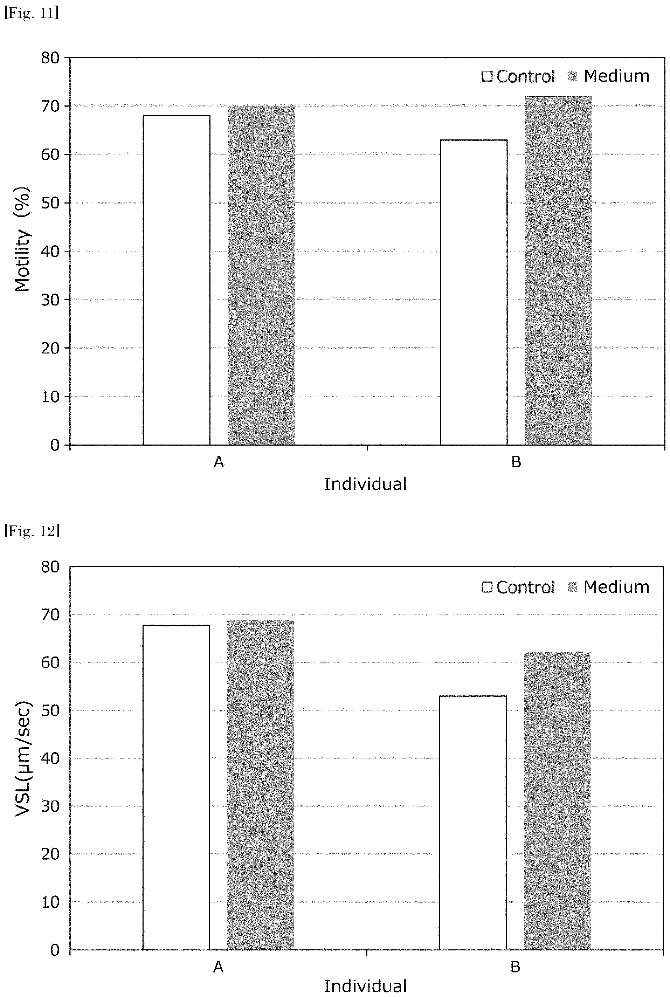

Fresh pig sperm on day 5 after ejaculation diluted with HIRO-SWINE B Solution were washed twice with HTF Medium (manufactured by FUJIFILM Wako Pure Chemical Corporation). The washed sperm were suspended in HTF Medium supplemented with RIM medium (serum-free medium for mesenchymal stem cells, Rohto, containing EGF, bFGF, albumin, transferrin, and insulin) in such a way as to have a final concentration of 10% (10% RIM medium) and HTF Medium supplemented with no medium as a negative control (control), and these were each used as a sample. Subsequently, the sample was cultured for 1 hour at 37°C. Next, 3 µL of the sample was analyzed by using a sperm motility analyzer (Computer Assisted Sperm Analysis (CASA) system). The proportion of motile sperm in all sperm observed (Motility, Fig. 11) and the straight-line velocity (VSL, Fig. 12) thereof were analyzed. It was confirmed that the supplementation with the mesenchymal stem cell medium increased the motility of the sperm in the pig.

### INDUSTRIAL APPLICABILITY

According to the present invention, sperm having high motility and suppressed sperm acrosome reaction can be provided. Sperm treated by the present invention have higher motility than and suppressed sperm acrosome reaction as compared with sperm treated by a conventional method, and thus have a remarkably higher fertilization success rate.

## Claims

1. A composition for treating sperm, comprising a medium for mesenchymal stem cells.

2. The composition for treating sperm according to claim 1, wherein the composition is a sperm conditioning solution, a sperm dilution solution, a sperm preservation solution, an artificial insemination solution, an in vitro fertilization solution, a solution for improving sperm motility, or a solution for retaining sperm fertility.

3. The composition for treating sperm according to claim 1 or 2, wherein the medium for mesenchymal stem cells is a serum-free medium.

4. The composition for treating sperm according to claim 3, wherein the medium for mesenchymal stem cells comprises at least one selected from the group consisting of EGF, bFGF, albumin, transferrin, and insulin.

5. An agent for improving sperm motility, comprising a medium for mesenchymal stem cells.

6. An agent for retaining sperm fertility, comprising a medium for mesenchymal stem cells.

7. A method for improving sperm motility, comprising culturing sperm in a solution comprising a medium for mesenchymal stem cells.

8. A method for retaining sperm fertility, comprising culturing sperm in a solution comprising a medium for mesenchymal stem cells.
